# EUROPÄISCHE PATENTANMELDUNG

(11) **EP 0 895 993 A1**
(43) Veröffentlichungstag der Anmeldung: **10.02.1999**
(21) Anmeldenummer: 98113973.6
(22) Anmeldetag: 25.07.1998
(51) Int. Cl.: C07D 295/04

(54) **Verfahren zur Herstellung von N-Aryl-N'-alkylpiperazinen**

(30) Priorität: 08.08.1997 DE 19734516
(71) Anmelder: BAYER AG, 51368 Leverkusen (DE)
(72) Erfinder: Komoschinski, Joachim, Dr., 51061 Köln (DE); Fiege, Helmut, Dr., 51373 Leverkusen (DE); Beitzke, Bernhard, Dr., 51503 Rösrath (DE)

(57) **Zusammenfassung**

N-Aryl-N'-alkyl-piperazine werden auf besonders einfache Weise, in guten Ausbeuten und in kurzen Reaktionszeiten erhalten, wenn man ein Anilin mit einem N-Alkyl-bishydroxyethyl-amin in Gegenwart einer starken Säure unter Druck umsetzt.

## Beschreibung

Die vorliegende Erfindung betrifft ein besonders vorteilhaftes Verfahren zur Herstellung von N-Aryl-N'-alkyl-piperazinen aus Anilinen und Diethanolaminen.

N-Aryl-N'-alkyl-piperazine sind wichtige Zwischenprodukte zur Herstellung von pharmazeutischen Wirkstoffen für diverse Indikationen. Beispielsweise kann man aus N-Aryl-N'-halogenpropyl-piperazinen durch Umsetzung mit s-Triazolo-[4,3-a]-pyridin-3-on Triazol-[4,3-a]-pyridinderivate mit sedierender, blutdruckerniedrigender und schmerzstillender Wirkung erhalten (siehe US-PS 3 381 009).

Es sind schon mehrere Verfahren zur Herstellung von N-Aryl-N'-alkyl-piperazinen bekannt geworden, die jedoch alle nicht befriedigend sind.

Gemäß der GB-PS 889 223 setzt man ein Anilinderivat zunächst mit Ethylenoxid zu dem entsprechenden N-Bishydroxyethyl-anilin um, tauscht dann dessen OH-Gruppen gegen Chlor aus und setzt abschließend das so erhaltene N-Bischlorethyl-anilin mit Ethanolamin unter Bildung eines Piperazinrings um. Dieses Verfahren ist vielstufig und deshalb aufwendig.

Die GB-PS 948 767 und die US-PS 3 326 916 beschreiben die Umsetzung von m-Trifluormethylanilin mit Diethanolamin/Bromwasserstoff bzw. Bis-2-chlorethylamin zu N-aryl-N'-unsubstituierten Piperazinen. Ein N'-Alkylsubstituent muß getrennt eingeführt werden. Auch dieses Verfahren ist mehrstufig und deshalb aufwendig. Hinzu kommt, daß die Handhabung von Bis-2-chlorethylamin und von Diethanolamin/Bromwasserstoff nur mit besonderem arbeitshygienischen Aufwand möglich ist.

Schließlich ist es bekannt, N-Aryl-N'-hydroxyethyl-piperazin durch Kondensation von Triethanolamin mit Anilin in Gegenwart von Schwefelsäure herzustellen, wobei man unter Sieden am Rückfluß, also drucklos arbeitet (siehe J.A.C.S. 58, 379(1936)). Erzielbare Ausbeuten sind nicht angegeben. Eine Nacharbeitung unter Einsatz von m-Trifluormethylanilin anstelle von Anilin hat ergeben, daß die erzielbaren Ausbeuten gering und die benötigten Reaktionszeiten lang sind (siehe Vergleichsbeispiel).

Es besteht deshalb noch immer das Bedürfnis nach einem einfachen Verfahren, mit dem N-Aryl-N'-alkyl-piperazine in guten Ausbeuten und kurzen Reaktionszeiten zugänglich sind.

Es wurde nun ein Verfahren zur Herstellung von N-Aryl-N'-alkyl-piperazinen gefunden, das dadurch gekennzeichnet ist, daß man ein Anilin mit einem N-Alkyl-bishydroxyethyl-amin in Gegenwart einer starken Säure unter Druck umsetzt.

Als Aniline kommen z.B. solche der Formel (I) infrage 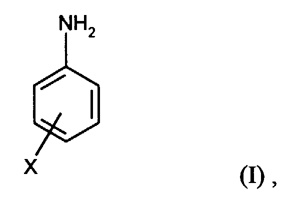 in der
- X: für Wasserstoff, Halogen, C₁-C₄-Alkyl, C₁-C₄-Halogenalkyl, C₁-C₄-Alkoxy, C₁-C₄-Halogenalkoxy oder Nitro steht.

Halogen, auch bei Halogenalkyl und Halogenalkoxy kann z.B. Fluor, Chlor oder Brom bedeuten.

Vorzugsweise steht X für Fluor, Chlor, Brom, Methyl, Ethyl, Trifluormethyl, Methoxy, Ethoxy, Trifluormethoxy oder Nitro. Besonders bevorzugt steht X für Trifluormethyl oder Methoxy.

Der Substituent X kann in o-, m- oder p-Position zur Aminogruppe angeordnet sein. Vorzugsweise befindet er sich in m-Position zur Aminogruppe.

Die Aniline können auch in Salzform eingesetzt werden, z.B. als Hydrochlorid oder Hydrosulfate.

Als N-Alkyl-bishydroxyethyl-amine kommen z.B. solche der Formel (II) infrage 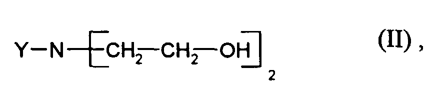 in der
- Y: für gegebenenfalls substituiertes C₁-C₄-Alkyl steht.

Als Substituenten für diese Alkylgruppen kommen z.B. Hydroxy- und Aminogruppen infrage, die vorzugsweise endständig angeordnet sind.

Vorzugsweise steht Y für Methyl, Ethyl, Isopropyl, 2-Hydroxyethyl, 3-Hydroxpropyl, 2-Aminoethyl oder 3-Aminopropyl.

Besonders bevorzugt steht Y für 2-Hydroxyethyl oder 3-Aminopropyl.

Auch die N-Alkyl-bishydroxyethyl-amine können gegebenenfalls in Salzform eingesetzt werden, z.B. als Hydrochloride oder Hydrosulfate.

Beim Einsatz von Anilinen der Formel (I) und von N-Alkyl-bis-hydroxyethyl-aminen der Formel (II) erhält man N-Aryl-N'-alkyl-piperazine der Formel (III) 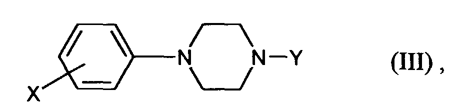 in der
- X und Y: die bei Formeln (I) und (II) angegebene Bedeutung haben.

Als starke Säuren kommen z.B. 85 bis 100 Gew.-%ige Schwefelsäure und Oleum mit SO₃-Gehalten bis zu 30 Gew.-% (bezogen auf H₂SO₄) infrage. Besonders bevorzugt ist 100 %ige Schwefelsäure, das sogenannte Monohydrat (von SO₃).

Das erfindungsgemäße Verfahren kann z.B. bei Drucken im Bereich 1,5 bis 15 bar durchgeführt werden. Bevorzugt sind 2 bis 12 bar.

Geeignete Drucke kann man auf verschiedene Weise einstellen. Beispielsweise kann man die Reaktionstemperatur so wählen, daß sich der gewünschte Druck im geschlossenen Gefäß von selbst einstellt. Man kann auch ein Inertgas, z.B. Stickstoff, Kohlendioxid, Helium oder Argon, in solchen Mengen aufdrücken, bis bei der jeweiligen Reaktionstemperatur der gewünschte Druck erreicht wird. Man kann weiterhin ein inertes verdampfbares Lösungsmittel zufügen und dann die Reaktionstemperatur so wählen, daß sich im Reaktor der gewünschte Druck einstellt. Hierfür geeignete Lösungsmittel sind beispielsweise geradkettige, verzweigte und cyclische gesättigte Kohlenwasserstoffe mit beispielsweise 5 bis 15 C-Atomen wie Pentane, Hexane, Heptane, Octane, Decane, Cyclopentan, Cyclohexan und Dekalin.

Als Reaktionstemperaturen kommen z.B. solche im Bereich von 130 bis 200°C, insbesondere von 140 bis 190°C, infrage.

Das Anilin der Formel (I) und das N-Alkyl-bishydroxyethyl-amin können z.B. in Molverhältnissen von 1:0,8 bis 1:3 eingesetzt werden. Bevorzugt liegt dieses Verhältnis bei 1:0,9 bis 1:1,1.

Bezogen auf 1 Mol eingesetztes Anilin der Formel (I) kann man z.B. 0,8 bis 2 Mole starke Säure einsetzen. Vorzugsweise liegt dieses Verhältnis bei 1:0,9 bis 1:1,3.

Wenn man zur Druckerzeugung ein inertes verdampfbares Lösungsmittel einsetzt, so kann man davon z.B. 5 bis 25 Vol.-%, bezogen auf das gesamte Reaktionsgemisch, einsetzen. Vorzugsweise beträgt diese Menge 10 bis 20 Vol.-%.

Die Reaktionszeit, d.h. die Zeit, bei der das Reaktionsgemisch nach dem Zusammenfügen der Reaktionspartner und der starken Säure unter Reaktionsbedingungen gehalten wird, kann z.B. 4 bis 20 Stunden, vorzugsweise 5 bis 12 Stunden betragen.

Das nach Beendigung der Reaktion vorliegende Gemisch kann man beispielsweise so aufarbeiten, daß man zunächst abkühlt und entspannt, dann mit einer Base neutralisiert, z.B. bis zu einem pH-Wert im Bereich 7 bis 11, ein organisches, mit Wasser nicht mischbares Lösungsmittel, z.B. eine aromatische Verbindung, zusetzt und die so erhaltene organische Phase abtrennt und destillativ aufarbeitet.

Mit der vorliegenden Erfindung kann man N-Aryl-N'-alkyl-piperazine in einem einstufigen Verfahren, in kurzen Reaktionszeiten und in guten Ausbeuten erhalten. Dies ist überraschend, denn die Anwendung von Druck bedingt höhere Reaktionstemperaturen. Bei höheren Reaktionstemperaturen ist aber mit Selektivitätsverminderung und der Bildung von Zersetzungsprodukten zu rechnen.

### Beispiele

### Beispiel 1

100 g 3-Trifluormethylanilin und 112,3 g Triethanolamin wurden in einen Autoklaven aus Tantal gefüllt und anschließend 85,2 g Schwefelsäure 100 %ig hinzugepumpt. Danach wurde der Autoklav verschlossen und 5 bar Stickstoff aufgedrückt. Der Autoklav wurde nun auf 170°C erhitzt, wobei sich ein Druck von 9,2 bar einstellte. Nach 8 h Reaktionsdauer wurde der Maximalumsatz zu N-2-Hydroxyethyl-N'-(3-trifluormethylphenyl)-piperazin erreicht. Das Reaktionsgemisch wurde abgekühlt und der Autoklav entspannt, danach die Mischung mit Wasser verdünnt und mit konz. Natronlauge auf pH 9 eingestellt. Danach extrahierte man 2 mal mit Toluol. Die so erhaltenen organischen Phasen wurden destillativ aufgearbeitet (10 cm Vigreux-Kolonne), wobei zunächst das Toluol abgezogen wurde. Es folgten als zweite Fraktion nicht umgesetztes 3-Trifluormethylanilin 63,4 g (Kp 48°C bei 2 mbar), welches für weitere Reaktionen einsetzbar ist, und als letzte Fraktion 51 g N-2-Hydroxyethyl-N'-(3-trifluormethylphenyl)-piperazin mit einem Siedebereich von 150° bis 195°C bei 2 mbar.

### Beispiel 2 (Vergleichsbeispiel)

### Arbeitsweise entsprechend J.A.C.S. 58, 379 (1936)

100 g 3-Trifluormethylanilin und 112,3 g Triethanolamin wurden in einem Rührkolben mit Rückflußkühler vorgelegt. Zu dieser Mischung wurden 85,2 g Schwefelsäure 100-%ig zugetropft und die Mischung zum Rückfluß erhitzt (Temperatur ca. 140°C). Bei dieser Reaktionsführung wurde der Maximalumsatz zu N-2-Hydroxyethyl-N'-(3-trifluormethylphenyl)-piperazin nach 30 h Reaktionszeit erreicht. Nach einer Reaktionszeit von 30 h wurde das Gemisch wie in Beispiel 1 beschrieben aufgearbeitet. Bei der Destillation wurden 65,1 g m-Trifluormethylanilin zurückgewonnen, während man 49,5 g N-2-Hydroxyethyl-N'-(3-trifluormethylphenyl)-piperazin erhielt.

### Beispiel 3

100 g 4-Trifluormethylanilin und 112,3 g Triethanolamin wurden in einen Autoklaven aus Tantal gefüllt und anschließend 85,2 g Schwefelsäure 100 %-ig hinzugepumpt. Danach wurde der Autoklav verschlossen und 5 bar Stickstoff aufgedrückt. Der Autoklav wurde nun auf 170°C erhitzt, wobei sich ein Druck von 9,5 bar einstellte. Nach 8 h Reaktionsdauer wurde der Maximalumsatz zu N-2-Hydroxyethyl-N'-(4-trifluormethylphenyl)-piperazin erreicht. Es wurde wie in Beispiel 1 beschrieben aufgearbeitet. Als zweite Fraktion wurde nicht umgesetztes 4-Trifluormethylanilin (62,9 g; Kₚ 51°C bei 2 mbar) und als letzte Fraktion 49,5 g N-2-Hydroxyethyl-N'-(4-trifluormethylphenyl)piperazin mit einem Siedebereich von 154° bis 198°C bei 2 mbar erhalten.

### Beispiel 4

130,2 g 3-Chloranilin und 180,8 g Triethanolamin wurden in einen Tantal-Autoklaven gefüllt und anschließend 138,6 g Schwefelsäure 100-%ig hinzugepumpt. Dann wurde der Autoklav verschlossen und auf 160°C erhitzt, wobei sich ein Eigendruck 3,3 bar aufbaute. Nach 16 h Reaktionsdauer wurde der Inhalt des Autoklaven wie in Beispiel 1 beschrieben aufgearbeitet. In diesem Fall destillierten nach der Toluol-Fraktion zunächst 20,64 g unverbrauchtes 3-Chloranilin ab (Kₚ 74° bis 78°C bei 1,4 bis 1,8 mbar) ab, welches wiederum eingesetzt werden konnte. Man erhielt anschließend 105,14 g N-2-Hydroxyethyl-N'-(3-chlorphenyl)-piperazin (Siedebereich 135° bis 188°C, bei 1,3 mbar), welches bei Raumtemperatur fest wurde.

### Beispiel 5

128 g 3-Chloranilin und 120 g N-Methyl-2,2'-iminodiethanol wurden in einen Tantal-Autoklaven vorgelegt und 119 g Schwefelsäure 100 %ig zugepumpt, danach mit 2 bar Stickstoff beaufschlagt und 15 h bei 160°C gehalten (Enddruck 4,9 bar). Die Aufarbeitung erfolgte wie in Beispiel 1 angegebe. Bei der Destillation wurden 69,27 g unverbrauchtes 3-Chloranilin (Kₚ 64° bis 74°C bei 1,1 bis 1,7 mbar), und danach 60,07 g N-Methyl-N'-(3-chlorphenyl)-piperazin (Kₚ 115° bis 130°C bei 1,7 mbar) erhalten.

### Beispiel 6

Auch in diesem Beispiel wurde die Reaktionsführung wie in Beispiel 1 beschrieben angewendet. Nun wurden 123 g 2-Methoxyanilin, 150 g Triethanolamin und 119 g Schwefelsäure 100 %ig eingesetzt und auf das Eindrücken von Stickstoff verzichtet. Nach 15 h Reaktionszeit bei einer Reaktionstemperatur von 150°C (Eigendruck ca. 2,8 bar) wurde wie in Beispiel 1 beschrieben, aufgearbeitet. Man erhielt bei der Destillation 16,16 g unverbrauchtes 2-Methoxyanilin (Kₚ 58° bis 91°C bei 1 mbar) und danach 99,89 g N-2-Hydroxyethyl-N'-(2-methoxyphenyl)-piperazin (Kₚ 95° bis 173°C bei 1 mbar).

### Beispiel 7

Hier kamen 123 g 2-Methoxyanilin, 120 g N-Methyl-2,2'-iminodiethanol und 119 g Schwefelsäure 100 %-ig zum Einsatz. Nachdem 2 bar Stickstoff aufgedrückt waren (Enddruck 4,8 bar) wurde der Autoklav 15 h bei 160°C gehalten. Die Aufarbeitung erfolgte auch hier wie in Beispiel 1 beschrieben. 52,71 g nicht umgesetztes 2-Methoxyanilin (Kₚ 74° bis 80°C bei 1,3 bis 1,7 mbar; Vigreux-Kolonne 10 cm) und 52,1 g N-Methyl-N'-(2-methoxyphenyl)-piperazin (Kₚ 113° bis 127°C bei 2,0 bis 2,2 mbar) wurden erhalten.

## Patentansprüche

1. Verfahren zur Herstellung von N-Aryl-N'-alkyl-piperazinen, dadurch gekennzeichnet, daß man ein Anilin mit einem N-Alkyl-bishydroxyethyl-amin in Gegenwart einer starken Säure unter Druck umsetzt.

2. Verfahren nach Anspruch 1, dadurch gekennzeichnet, daß man ein Anilin der Formel (I) einsetzt, 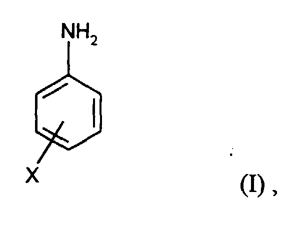 in der
X für Wasserstoff, Halogen, C₁-C₄-Alkyl, C₁-C₄-Halogenalkyl, C₁-C₄-Alkoxy, C₁-C₄-Halogenalkoxy oder Nitro steht,
ein N-Alkyl-bishydroxyethyl-amin der Formel (II) einsetzt 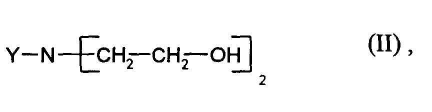 in der
Y für gegebenenfalls substituiertes C₁-C₄-Alkyl steht
und ein N-Aryl-N'-alkyl-piperazin der Formel (III) erhält 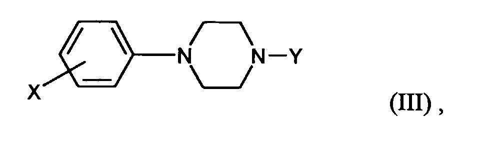 in der
X und Y die bei Formeln (I) und (II) angegebene Bedeutung haben.

3. Verfahren nach Ansprüchen 1 und 2, dadurch gekennzeichnet, daß man als starke Säure 85 bis 100 gew.-%ige Schwefelsäure oder Oleum mit einem SO₃-Gehalt bis zu 30 Gew.-% (bezogen auf H₂SO₄) einsetzt.

4. Verfahren nach Ansprüchen 1 bis 3, dadurch gekennzeichnet, daß man bei Drucken im Bereich 1,5 bis 15 bar arbeitet.

5. Verfahren nach Ansprüchen 1 bis 4, dadurch gekennzeichnet, daß die Reaktionstemperatur im Bereich von 130 bis 200°C liegt.

6. Verfahren nach Ansprüchen 1 bis 5, dadurch gekennzeichnet, daß man das Anilin und das N-Alkyl-bishydroxyethyl-amin in Mol-Verhältnissen von 1:0,8 bis 1:3 einsetzt.

7. Verfahren nach Ansprüchen 1 bis 6, dadurch gekennzeichnet, daß man pro Mol eingesetztes Anilin 0,8 bis 2 Mole starke Säure einsetzt.

8. Verfahren nach Ansprüchen 1 bis 7, dadurch gekennzeichnet, daß die Reaktionszeit 4 bis 20 Stunden beträgt.

9. Verfahren nach Ansprüchen 1 bis 8, dadurch gekennzeichnet, daß man das nach Beendigung der Reaktion vorliegende Gemisch aufarbeitet, indem man es zunächst abkühlt und entspannt, dann mit einer Base neutralisiert, ein organisches, mit Wasser nicht mischbares Lösungsmittel zusetzt und die so erhaltene organische Phase abtrennt und destillativ aufarbeitet.
